# EUROPÄISCHE PATENTSCHRIFT

(11) **EP 0 604 830 B1**
(45) Veröffentlichungstag und Bekanntmachung des Hinweises auf die Patenterteilung: **30.09.1998**
(21) Anmeldenummer: 93120190.9
(22) Anmeldetag: 15.12.1993
(51) Int. Cl.: A61K 31/00, A61K 31/55, A61K 31/58, A61K 31/685, A61K 31/425

(54) **Verwendung mindestens eines Paf-Antagonisten zur Herstellung eines Arzneimittels zur Behandlung oder Vorbeugung des Hyperinsulinismus**
Use of at least one PAF-antagonist for the manufacture of a medicament in the treatment or prevention of hyperinsulinism
Utilisation d'au moins un antagoniste du PAF dans la fabrication d'un médicament pour le traitement ou la prévention de l'hyperinsulinisme

(30) Priorität: 28.12.1992 DE 4244265
(43) Veröffentlichungstag der Anmeldung: 06.07.1994
(73) Patentinhaber: Korth, Ruth-Maria, Dr. med, D-80639 München (DE)
(72) Erfinder: Korth, Ruth-Maria, Dr. med, D-80639 München (DE)
(74) Vertreter: Vossius, Volker, Dr.

(56) Entgegenhaltungen:
- EP-A- 0 255 028
- EP-A- 0 312 913
- DE-A- 3 710 921
- DIABETIC MEDECINE, Bd. 7, 1990 Seiten 414-9, TROVATI ET AL 'Desensitization of the Platelet Aggregation Response to Adrenaline During Insulin-induced Hypoglycemia in Man'
- CIRCULATION, Bd. 72, 1985 Seiten 718-27, ZIMMERMAN ET AL 'Production of Platelet-activating Factor by Human Vascular Endothelial Cells: Evidence for a Requirement for Specific Agonists and Modulation by Prostacyclin'
- EUR. J. PHARMACOL., Bd. 156, 1988 Seiten 207-14, LANG ET AL 'Attenuation of Burn-induced Changes in Hemodynamics and Glucose Metabolism by the PAF Antagonist SRI 63-675'
- BIOCHEM. J., Bd. 269, 1990 Seiten 269-72, EVANS ET AL 'Metabolic Effects of Platelet-activating Factor in Rats in vivo'
- BR. J. PHARMACOL., Bd. 98, 1989 Seiten 653-61, KORTH ET AL 'Interaction of the PAF Antagonist WEB2086 and its Hetrazepine Analogues with Human PLatelets and Endothelial Cells'
- EUR. J. PHARMACOL., Bd. 123, 1986 Seiten 197-05, NUNEZ ET AL 'Specific Inhibition of PAF-acether-induced Platelet Activation by BN52021 and Comparison with the PAF-acether Inhibitors Kadsurenone and CV3988'
- Molekularbiologie der Zelle (1987), S.799,1007

## Beschreibung

Die Erfindung betrifft die neue Ausbildung von Bindungstellen (Rezeptoren) für Paf und seiner Analoga an endothelialen Zellen und endothelialen Zellinien. Da die endothelialen Paf-Bindungsstellen z.B. durch Insulin ausgebildet werden, betrifft die Erfindung die Verwendung von Paf-Antagonisten zur Herstellung eines Arzneimittels zur Vorbeugung und Therapie des Hyperinsulinismus. Die Erfindung betrifft somit die Verwendung von Paf-Antagenisten zur Herstellung von Medikamenten, die einer neuen Ausbildung von Paf-Bindungsstellen auf endothelialen Zellen und Zellinien entgegenwirken oder die neu ausgebildeten Paf-Bindungsstellen hemmen.

Die chemische Struktur von Paf und seinen Analoga ist bekannt als 1-Alkyl-2-acetyl-sn-glyceryl-3-phosphorylcholin (J. Exp. Med. 136: 1356-1377, 1972; J. Biol. Chem., 254: 9355-9358, 1979). Es ist auch bekannt, daß Paf und seine Analoga starke Entzündungs- und Gefäßreaktionen hervorrufen können. Paf-Rezeptoren wurden an verschiedenen Blutzellen und an Endothelzellen beschrieben, wobei sich die Paf-Bindungsstellen von Blut- und Endothelzellen unterscheiden (DE 3710921 A1, 14.7.88). Es wurde auch gezeigt, daß eine kurze Zellaktivierung mit Phorbolestern und eine Langzeitaktivierung mit Lipiden und Lipoproteinen z.B. an monozytären Zellinien spezifische Paf-Bindungsstellen ausbilden (Chem. Phys. Lipids 59, 207-213, 1991). Das gen der Paf-Bindungsstellen an Blutzellen ist bekannt (Nature 349, 342-346, 1991), während das gen der endothelialen Bindungsstellen bisher nicht definiert wurde.

In der deutschen Patentoffenlegungsschrift DE 37 10 921 wird die Behandlung von Erkrankungen, die durch die Herabsetzung der Endothelzellenbarriere hervorgerufen werden, beschrieben; siehe Spalte 1, Zeile 44 bis 49.

In der europäischen Patentoffenlegungsschrift EP-A3-0 312 913 wird die Vorbeugung und Behandlung von Erkrankungen mit Paf-Acether-Antagonisten beschrieben, die durch den Abbau der Endothelzellenbarriere hervorgerufen werden; siehe Seite 2, Zeile 22 bis 29.

In Travoti et al., *Diabetic Medicine,* 7 (1990), 414-419 wird der Einfluß der Insulininduzierten Unterzuckerung im Blut (Hypoglykämie) auf die Plättchensensitivität gegenüber Adrenalin und nichtadrenergen Agonisten im Menschen beschrieben; siehe Seite 414, Zusammenfassung, Zeile 1 und 2. Es wird gezeigt, daß während der Insulininduzierten Unterzuckerung im Blut ein Anstieg der Plättchensensitivität gegenüber nichtadrenergen Agonisten, unter anderem Paf, und ein Abfall der Plättchenantwort gegenüber Adrenalin vorliegt; siehe Seite 414, Zusammenfassung, Zeile 13 bis 18 und Seite 417 und 418, Diskussion, Zeile 1 bis 4. Ferner wird die Sensitivität von Thrombozyten untersucht.

In Zimmerman et al., *Circulation,* 72 (1988), 718-726 wird die Synthese von Paf in kultivierten Endothelzellen aus Nabelschnurvenen beschrieben. Hoch gereinigtes menschliches Thrombin und die Calcium-Ionophore A23187 stimulieren die Paf-Synthese; siehe Seite 718, Zusammenfassung, Zeile 1 bis 5. Eine Behandlung mit Insulin zeigt keinen Einfluß auf die Synthese von Paf; siehe Seite 722, Tabelle 1.

In Evans et al., *Biochem. J.*, 269 (1990), 269-272 werden die Wirkungen von Paf auf den Leberstoffwechsel in Ratten untersucht; siehe Seite 269, Zusammenfassung, Zeile 1 und 2. Hohe Paf-Dosierungen verringern das Leberglykogen; siehe Seite 269, Zusammenfassung, Zeile 4. Alle Paf-Dosierungen, bis auf die geringste eingesetzte, verringern den Plasma-Insulin-Gehalt mit gleichzeitigem Anstieg des Blutzuckers; siehe Seite 269, Zusammenfassung, Zeile 4 und 5. Es wird diskutiert, daß Streßhormone eine verminderte periphere Glukoseverwertung hervorrufen, die zu einer Absenkung des Insulins und einem Blutzuckeranstieg führt; siehe Seite 270, rechte Spalte, dritter Absatz.

In Korth et al, *Br.J. Pharmacol.* 98 (1989), 653-661 wird die Hemmung der Bindung von Paf-Acether an Blutplättchen und Nabelschnur-Endothelzellen sowie der Paf-vermittelte Calciumfluß durch WEB 2086, 2098, 2105 und 2118 untersucht; siehe Seite 653, Zusammenfassung, Punkte 1 bis 6. Es wird jedoch nicht die Wirkung von Paf oder Paf-Antagonlsten auf differenzierte endotheliale Zellen beschrieben.

Der Erfindung liegen endotheliale Zellen und endotheliale Zellinien, die kaum Paf-Bindungsstellen aufweisen, zugrunde. Uberraschender Weise wurde gefunden, daß z.B. der Wachstumsfaktor Insulin neue endotheliale Paf-Bindungsstellen ausbilden kann. Auf diesem Befund basiert die Erfindung und betrifft somit die Verwendung von Paf-Antagonisten zur Herstellung von Medikamenten, die z.B. Schäden durch einen Hyperinsulinismus verhindern oder ihnen vorbeugen sollen. Wenn z.B. Insulin endotheliale Paf-Bindungsstellen ausbildet, so wird durch Insulin die Phagozytose und Durchschleusung von Blutzellen einschließlich Viren, Bakterien gefördert und schädlich Stoffe sowie einweißreiche Blutflüßigkeit und Lipide im subendothelialen Raum angereichert. Dieses könnte z.B. im Falle des Hyperinsulinismus zu Gefäßschäden führen, die erst Jahre danach auftreten und kaum kausal zu behandeln sind, da sie vorzugsweise die kleinen Gefäße z.B. des Auges, der Niere, des Herzens, des Gehirns und der Peripherie betreffen. Außerdem entstehen im schlecht durchbluteten Gewebe saure pH-Werte, wie ebenfalls gefunden wurde, die neue Ausbildung von endothelialen Paf-Bindungstellen fördern. Die Erfindung betrifft somit die Verwendung von Paf-Antagonisten, die der neuen Ausbildung von endothelialen Paf-Bindungsstellen entgegen wirken sollen oder neu ausgebildete endotheliale Paf-Bindungsstellen hemmen sollen.

Die neue Ausbildung der endothelialen Paf-Bindungsstellen kann in der Weise therapeutisch beeinflußt werden, daß eine neue Ausbildung von endothelialen Paf-Bindungsstellen z.B. durch Insulin verhindert wird oder neu ausgebildete endotheliale Paf-Bindungsstellen blockiert werden. Es können beliebige Substanzen untersucht werden, z.B. Hormone wie Östrogene, die der neuen Ausbildung von endothelialen Paf-Rezeptoren entgegenwirken sollen und somit als Schutz gegen Gefäßschäden z.B. beim Hyperinsulinismus dienen. An Stelle des Insulins können z.B. auch andere Wachstumsfaktoren, Hormone, Mediatoren, Proteine oder Lipide, Mikroorganismen, jegliche Endothelreizung, Endothelschädigung und Endotheldifferenzierung einschließlich saurer pH-Werte neue endotheliale Paf-Bindungsstellen ausbilden, die dann gehemmt werden müssen bzw. deren Ausbildung vorgebeugt werden sollte.
Die Substanzen, die neu ausgebildete endotheliale Paf-Bindungsstellen hemmen können, sind Triazolothieno-diazepine mit Homologen, Ginkgolide und Paf-Analogua, z.B. CV 3988 (Life Science 1983, 32, S.263). Triazolothieno-diazepine sind im Br. J. Pharmacol. 1987, 90, S. 139, Ginkgolide im "Blood and Vessel" 1985, 16, S. 558 beschrieben. Triazolothieno-diazepine-Substanzen können z.B. WEB 2086 and WEB 2098 sein. Als Ginkgolide können z.B. BN 52020, BN 52021 und BN 52063, das im Eur. J. Pharmacol. 1988, 152, s. 101 beschrieben wurde, benützt werden. Die chemische Substanz BN 50739, die im J. Pharmacol. Exp. Therapeutics, 1990, 254, S.976 beschrieben wurde, könnte ebenfalls zur Behandlung genützt werden.
Die chemische Bezeichnung für CV 3988 ist rac-3-(N-n-Octadecyl-carbamoyloxy)-2-methoxypropyl-2-tiazolioethyl phosphat; die chemische Bezeichnung für WEB 2086 ist 3-(4-(2-Chlorphenyl)-9-methyl-6H-thieno[3,2-f](1,2,4) triazolo[4,3-a](1,4)-diazepine-2-yl)-1-(4-morpholinyl)-1-propanon; die chemische Bezeichnung für WEB 2098 ist (3-(4-(2-Chlorphenyl)-9-cyclopropyl-6H-thieno[3,2-f](1,2,4)-triazolo[4,3-a](1,4)-diazepin-2-yl)-1-(4-morpholinyl)-1-propanon; die chemische Bezeichnung für BN 52020 ist 9H-1,7a-(Epoxymethano)-1H,6aH-cyclopenta[c]furo[2,3-b]furo[3 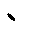 ,2 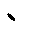 : 3,4]cyclopenta[1,2-d]furan-5,9,12-(4H)-trion-3-tert-butylhexahydro-4, 7b-dihydroxy-8-methyl; die chemische Bezeichnung für BN 52021 ist 9H-1,7a-(Epoxymethano)-1H,6aH-cyclopenta[c]furo[2,3-b]furo[3 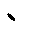 ,2 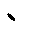 :3,4] cyclopenta[1,2-d]furan-5,9,12-(4H)-trion-3-tert-butylhexahydro-4,7b-11-trihydroxy-8-methyl; die chemische Bezeichnung für BN 52022 ist 9H-1,7a-(Epoxymethano)-1H,6aH-cyclopenta[c]furo[2,3-b]furo[3 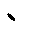 ,2 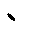 :3,4]cyclopenta[1,2-d] furan-5,9,12-(4H)-trion-3-tert-butylhexahydro-2,4,7b,11-tetrahydroxy-8-methyl. Die chemische Bezeichnung für BN 50739 ist Tetrahydro-4,7,8,10 methyl-(chlor-2-phenyl)-6-[(dimethoxy-3,4-phenyl)-thio] methylthiocarbonyl-9-pyrido[4',3'-4,5]thieno[3,2-f]triazolo-1,2,4[4, 3-a]diazepine-1,4.
Die Paf-Antagonisten können örtlich, oral, parenteral oder durch Inhalation verabreicht werden. Die Substanzen können in allen pharmakologischen Darreichungsformen gegeben werden, als Ingredentien in konventionellen pharmazeutischen Präparaten, z.B. als Tabletten, Dragees, Kapseln, Puder, Pastillen, Zäpfchen, Salben, Pasten, Lotionen, Emulsionen, Lösungen, Suspensionen, Aerosole, Sirup und in Liposomen u.s.w., d.h. alle Arten, die eine pharmazeutische Trägersubstanz mit effektiven Dosen der Substanzen verbindet. Die effektive Dosis wurde dabei als 10 bis 500 oder 0,1 and 50 mg per Dosis für intravenöse oder intramuskuläre Verabreichung vorgeschlagen.

Um einer einfachen Test bereitzustellen, der die neue Ausbildung von endothelialen Paf-Bindungsstellen untersucht, d.h. um ein Screening-Verfahren für Paf-Antagonisten durchzuführen, wird das folgende Verfahren vorgeschlagen:
a) Endotheliale Zellen oder Zellinien werden kultiviert,
b) die endothelialen Zellen werden durch Kultur in Anwesenheit von z.B. Insulin über einen längeren Zeitraum z.B. 2 bis 24 Std. stimuliert,
c) die endothelialen Zellen werden vorzugsweise mit sauren pH-Werten gewaschen und Bindungsstudien mit Paf, Paf-Analoga, Paf-Antagonisten und/oder Antikörpern werden wie beschrieben (DE 37 10921 A1, 14.7.1988) durchgeführt, wobei entprechend zu Schritt b) die stimulierten und/oder die nicht stimulierten endothelialen Zellen in Anwesenheit und Abwesenheit von markierten und nicht markierten Agonisten und/oder Antagonisten verglichen werden.
d) Sodann wird entsprechend c) das Erbgut der stimulierten endothelialen Zellen definiert, angereichert und auf lösliche Zellen übertragen.

Die Kultur von endothelialen Zellen und/oder Zellinien wird z.B. wie bereits beschrieben mit beliebigen Standardtechniken durchgeführt (DE 3710921 A1). Dabei können die endothelialen Zellen und Zellinien z.B. in immortalisierter Form verwendet werden. An Stelle von Kulturschalen können die endothelialen Zellen auch auf löslichen Partikeln kultiviert werden, um Lösungschritte von adhärenten endothelialen Zellen zu vereinfachen. Die Kulturschalen und löslichen Partikel können auch vorbehandelt werden, z.B. mit einem Gemisch aus Gelatine und/oder Wachstumsfaktoren, Hormonen, Mediatoren, Lipiden und/oder Hemmstoffen.

Die endothelialen Zellen und Zellinien können auch auf anderen Zellen z.B. glatten Muskelzellen zum Wachstum gebracht werden, um die zelluläre Interaktion mit glatten Muskelzellen, die zur Gefäßregulierung führt, besser zu definieren und zu behandeln. In diesem Zusammenhang können auch beliebige funktionelle Tests z.B. der Einstrom von Calcium oder eine Mediatorproduktion und/oder Freisetzung in Anwesenheit von Paf-Antagonisten oder Antikörpern verwendet werden.
Um die Ausbildung von Paf-Rezeptoren z.B. durch Insulin zu untersuchen werden entsprechend Schritt c) die Bindungsstudien nach einer Kultur in Anwesenheit von z.B. 2 I.E. Humaninsulin durchgeführt, wobei Insulin z.B. den Standardmedien vorzugweise 2 bis 24 Std. beigefügt wird. Es können auch andere Insulinarten und/oder andere Wachstumsfaktoren und Substanzen beigefügt werden, einschließlich pharmakologischer Substanzen, die die neue Ausbildung der endothelialen Paf-Bindungsstellen hemmen sollen oder bereits ausgebildete endotheliale Paf-Bindungsstellen hemmen. Die Paf-Bindungsstudien werden z.B. wie bereits beschrieben durchgeführt (DE 37 10921 A1). Es können markierte Paf-Agonisten und/oder markierte Paf-Antagonisten sowie Antikörpern zusammen mit unmarkierten Substanzen benützt werden.

### Beispiel:

Eine endotheliale Zellinie, die kaum endotheliale Paf-Bindungsstellen aufweist, wird in Anwesenheit von 2 I.E. Human insulin (Hoechst, Deutschland) in der Weise differenziert, daß sich endotheliale Paf-Bindungsstellen neu ausbilden. Die adhärenten, konfluenten Endothelzellen binden ohne Stimulation mit Insulin nur 6,8 fmol radioaktiv markiertes Paf per Kulturschale konfluenter, adhärenter endothelialer Zellen und dieses radioaktiv markierte Paf wird durch 500 nM unmarkiertes Paf nicht aus der Bindung verdrängt. Im Gegenteil, die totale Bindung von radioaktiv markiertem Paf beträgt in Anwesenheit von unmarkiertem Paf sogar 9,6 fmol. D.h. unmarkiertes Paf stimuliert und hemmt nicht die Bindung von radioaktiv markiertem Paf und das beweist, daß diese Zellinie ohne Behandlung keine endothelialen Paf-Bindungsstellen auf ihrer Oberfläche haben. Dagegen binden die endothelialen Zellen nach Langzeitkultur (20 Std) in Anwesenheit von 2 I.E. Humaninsulin 68 fmol radioaktiv markiertes Paf per Kulturschale adhärenter, konfluenter endothelialer Zellen, wobei 50 nM unmarkiertes Paf die Bindung auf 53,7 fmol und 500 nM die Bindung auf 28,1 fmol senken. Dadurch wird nach einer Kultur in Anwesenheit von 2 I.E. Insulin per Kulturschale adhärenter, konfluenter endothelialer Zellen eine spezifische endotheliale Bindung von radioaktiv markiertem Paf erreicht, die ohne Insulin nicht vorhanden ist (Tabelle 1). Mit anderen Worten, die untersuchte endotheliale Zellinie weist nur nach Kultur in Anwesenheit von Insulin spezifische endotheliale Paf-Bindungsstellen auf. Die endothelialen Paf-Bindungsstellen werden also durch eine Kultur in Anwesenheit von z.B. Insulin neu ausgebildet und bestehen vorher nicht. Auch ein Paf-Antagonist z.B BN 50739 (10 und 20 µM) hat keinen Einfluß auf die Bindung von markiertem Paf, wenn diese endotheliale Zellinie nicht stimuliert wird. Erfindungsgemäß entwickelt BN 50739 (20 µM) aber eine spezifische Bindung von 5,8 fmol per Kulturschale adhärenter, konfluenter endothelialer Zellen, wenn die endothelialen Zellen 20 Std. in Anwesenheit von 2 I.E. Insulin kultiviert wurden, denn dadurch werden endotheliale Paf-Bindungsstellen neu ausgebildet.

### Tabelle 1:

Bindung von radioaktiv markiertem Paf (0,69 nM, 30 Min, 20 ° C) an A) unbehandelte endotheliale Zellen und B) nach Kultur (20 Std.) mit 2 I.E. Insulin per Kulturschale adhärenter, konfluenter endothelialer Zellen. Die Ergebnisse stellen fmol der Bindung von radioaktiv markiertem Paf an adhärente, konfluente endotheliale Zellen per Kulturschale (50 cm²) dar und sind representativ für 3 gleiche Ergebnisse.

| Totale Bindung | Unspezifische Bindung in Anwesenheit von nicht markiertem Paf | Spezifische Bindung |
|---|---|---|
| | | |

| A) Ohne Insulin: | | |
|---|---|---|
| | 500 nM Paf: | |
| 6,8 fmol | 9,6 fmol | keine |

| B) 2 I.E. Insulin für 20 Std. | | |
|---|---|---|
| | 50 nM Paf: | |
| 68 fmol | 53,7 fmol | 14,7 fmol |
| | 500 nM Paf: | |
| 68 fmol | 28,1 fmol | 39,9 fmol |

D.h. ohne Insulin findet sich erfindungsgemäß keine spezifische endotheliale Paf-Bindung, während nach einer Kultur in Anwesenheit von 2 I.E. Insulin (20 Std.) 14,7 fmol und 39,9 fmol [³H]Paf in spezifischer Weise an konfluente, adhärente endotheliale Zellen gebunden wird

## Patentansprüche

1. Verwendung mindestens eines Paf-Antagonisten zur Herstellung eines Arzneimittels zur Behandlung oder Vorbeugung des Hyperinsulinismus.

2. Verwendung nach Anspruch 1, wobei ein Paf-Antagonist ein Ginkgolid oder Triazolothieno-diazepin ist.

3. Verwendung nach Anspruch 2 wobei das Ginkgolid BN 52020, BN 52021 oder BN 52063 und das Triazolothieno-diazepin WEB 2086, WEB 2098 oder BN 50739 ist.

4. Verwendung nach Anspruch 1 oder 2, wobei das Arzneimittel örtlich, oral, parenteral oder durch Inhalation, z. B. als Tablette, Dragee, Kapsel, Puder, Pastille, Zäpfchen, Salbe, Paste, Lotion, Emulsion, Lösung, Suspension, Aerosol, Sirup oder Liposom verabreicht werden kann.

## Claims

1. The use of at least one paf antagonist for the manufacture of a medicament for the treatment or prevention of hyperinsulinemia.

2. The use according to claim 1, wherein a paf antagonist is a ginkgolide or a triazolothieno-diazepine.

3. The use according to claim 2, wherein the ginkgolide is BN 52020, BN 52021 or BN 52063 and the triazolothieno-diazepine is WEB 2086, WEB 2098 or BN 50739.

4. The use according to claim 1 or 2, wherein the medicament can be administered topically, orally, parenterally or by inhalation, for example as a tablet, coated tablet, capsule, powder, lozenge, suppository, creme, ointment, lotion, emulsion, solution, suspension, aerosol, syrup or liposome.

## Revendications

1. Utilisation d'au moins un antagoniste de PAF pour la préparation d'un médicament pour le traitement ou la prévention de l'hyperinsulinémie.

2. Utilisation selon la revendication 1, dans laquelle un antagoniste de PAF est un ginkgolide ou une triazolothiéno-diazépine.

3. Utilisation selon la revendication 2, dans laquelle le ginkgolide est BN 52020, BN 52021 ou BN 52063 et la triazolothiéno-diazépine est WEB 2086, WEB 2098 ou BN 50739.

4. Utilisation selon la revendication 1 ou 2, dans laquelle le médicament peut être administré par voie topique, orale, parentérale ou par inhalation, par exemple sous forme de comprimé, comprimé enrobé, capsule, poudre, pastille, suppositoire, crème, pommade, lotion, émulsion, solution, suspension, aérosol, sirop ou liposome.
